**Europäisches Patentamt**

**European Patent Office**

**Office européen des brevets**

(19)

(11) Veröffentlichungsnummer : **0 355 350 B1**

(12)

# EUROPÄISCHE PATENTSCHRIFT

(45) Veröffentlichungstag der Patentschrift :
**25.11.92 Patentblatt 92/48**

(51) Int. Cl.$^5$ : **C07C 45/74,** C07C 45/62,
C07C 45/73, C07C 49/233,
C07C 49/235

(21) Anmeldenummer : **89112402.6**

(22) Anmeldetag : **07.07.89**

(54) **Verfahren zur Herstellung von 4,4-Dimethyl-l-(p-chlorphenyl)-pentan-3-on.**

(30) Priorität : **20.07.88 DE 3824518**

(43) Veröffentlichungstag der Anmeldung :
**28.02.90 Patentblatt 90/09**

(45) Bekanntmachung des Hinweises auf die
Patenterteilung :
**25.11.92 Patentblatt 92/48**

(84) Benannte Vertragsstaaten :
**BE CH DE FR GB IT LI NL**

(56) Entgegenhaltungen :
**EP-A- 0 108 236**
**FR-A- 2 253 505**
**GB-A- 1 595 699**

(73) Patentinhaber : **BAYER AG**
**W-5090 Leverkusen 1 Bayerwerk (DE)**

(72) Erfinder : **Mais, Franz-Josef, Dr.**
**Gustav-Poensgen-Strasse 23**
**W-4000 Düsseldorf 1 (DE)**
Erfinder : **Fiege, Helmut, Dr.**
**Walter-Felx-Strasse 23**
**W-5090 Leverkusen 1 (DE)**
Erfinder : **Henneke, Karl-Wilhelm, Dr.**
**Heymannstrasse 38**
**W-5090 Leverkusen 1 (DE)**

## Beschreibung

Die vorliegende Erfindung betrifft ein Verfahren zur Herstellung von 4,4-Dimethyl-1-(p-chlor-phenyl)-pentan-3-on durch Kondensation von Pinakolon mit p-Chlorbenzaldehyd zu 4,4-Dimethyl-1-(p-chlor-phenyl)-1-penten-3-on und dessen anschließende Hydrierung. Das Verfahrensprodukt dient als Zwischenprodukt zur Herstellung von Wirkstoffen im Pflanzenschutz und im Pharmasektor (DE-OS 27 37 489, DE-OS 33 15 510, DE-OS 33 15 681).

Die Kondensation von Pinakolon mit substituierten Benzaldehyden ist bekannt (Arch. Pharm. (Weinheim) 311 (1978), 604; Tetrahedron 31 (1975), 3); sie führt zu 4,4-Dimethyl-1-aryl-1-penten-3-onen. In der DE-OS 32 37 476 wird in Beispiel 1b eine Verfahrensweise beschrieben, nach der p-Chlorbenzaldehyd und Pinakolon in Gegenwart von Natriumhydroxid in Ethanol 12 Stunden unter Rückfluß erhitzt werden, dann das Lösungsmittel Ethanol abgedampft wird und der Rückstand in Wasser/ Methylenchlorid aufgenommen wird. Danach wird angesäuert und dann das Pentenon aus der Methylenchloridphase durch Eindampfen isoliert. Die Ausbeute beträgt dort nur 59 % an Rohprodukt. Nachteilig ist neben der schlechten Ausbeute die umständliche wäßrige Aufarbeitung. In der DE-OS 27 37 489 ist eine Verfahrensweise beschrieben, gemäß der p-Chlorbenzaldehyd mit Pinakolon in Gegenwart von Natriumhydroxid in Methanol/Ethanol bei Temperaturen bis maximal 25°C umgesetzt wird. Das 4,4-Dimethyl-1-(p-chlor-phenyl)-1-penten-3-on fällt in fester Form an, muß abgesaugt und mit weiterem Lösungsmittel gewaschen werden. Die erforderliche niedrige Temperatur bedingt eine inhomogene, zum Teil feste Reaktionsmischung, die nur schwer rührbar ist. Weiterhin müssen molare Mengen an Natriumhydroxid eingesetzt werden. Nachteilig ist weiterhin, daß die in der Mutterlauge verbleibenden Anteile an Reaktionsprodukt in einem zusätzlichen Aufarbeitungsschritt isoliert werden müssen. Zur Hydrierung der Doppelbindung wird das Pentenon in Ethylacetat gelöst und mit etwa 26 Gew.-% Raney-Nickel bei Atmosphärendruck und einer Reaktionsdauer von etwa 14 1/2 Stunden mit Wasserstoff umgesetzt. Die sehr große Katalysatormenge und die sehr lange Reaktionszeit sind die entscheidenden Nachteile dieses Verfahrens. In allen bekannten Verfahrensweisen muß das Kondensationsprodukt von Pinakolon und p-Chlorbenzaldehyd als Feststoff isoliert und gereinigt werden und wird erst danach in einer zweiten getrennten Operation der Hydrierung zugeführt.

Es wurde nun ein Verfahren zur Herstellung von 4,4-Dimethyl-1-(p-chlor-phenyl)-pentan-3-on durch Kondensation von Pinakolon und p-Chlorbenzaldehyd in einem Alkohol als Lösungsmittel in Gegenwart einer anorganischen Base und anschließende Hydrierung gefunden, das dadurch gekennzeichnet ist, daß man

a) das bei der Kondensation erhaltene Reaktionsgemisch direkt ohne Zwischenisolierung des 4,4-Dimethyl-1-(p-chlor-phenyl)-1-penten-3-ons nach Zugabe eines Hydrierkatalysators bei erhöhter Temperatur und überatmosphärischem Druck zum 4,4-Dimethyl-1-(p-chlor-phenyl)-pentan-3-on hydriert,

b) nach Abtrennung des Hydrierkatalysators vom flüssigen Hydriergemisch den Alkohol weitgehend abdestilliert und den Wassergehalt des Destillationssumpfes so einstellt, daß er sich in eine wäßrige und eine organische Phase trennt und

c) das 4,4-Dimethyl-1-(p-chlor-phenyl)-pentan-3-on aus der organischen Phase gewinnt.

Erfindungsgemäß können beliebige Verhältnisse von Pinakolon und p-Chlorbenzaldehyd umgesetzt werden. Zur Vermeidung der Aufarbeitung unnötig großer Überschüsse werden im allgemeinen 1-1,1 Mol Pinakolon pro Mol p-Chlorbenzaldehyd eingesetzt.

Als anorganische Base kommen die Hydroxide oder Carbonate von Alkali- oder Erdalkalimetallen in Frage, beispielsweise Natriumhydroxid, Kaliumhydroxid, Magnesiumhydroxid, Kalziumhydroxid, Natriumcarbonat, Kaliumcarbonat; bevorzugt werden Natrium- oder Kaliumhydroxid eingesetzt. Als Basen kommen weiterhin die Oxide der genannten Elemente in Frage, die unter den Reaktionsbedingungen die Hydroxide oder Carbonate bilden können.

Die Menge der zuzusetzenden Base kann in weiten Grenzen schwanken. Es wurde jedoch gefunden, daß 5-30 Äquivalent-%, bevorzugt 10-15 Äquivalent-% Base, bezogen auf die Menge an p-Chlorbenzaldehyd, ausreichend sind und somit einen unnötig hohen Verbrauch an Base vermeiden helfen.

Die Basen können sowohl als solche als auch in gelöster Form dem Reaktionsgemisch zugeführt werden. Bevorzugt ist der Einsatz als gelöste Form.

Als Alkohole können ein- oder mehrwertige eingesetzt werden; bevorzugt sind einwertige Alkohole. Alkohole mit zu großer Alkyl- oder Arylgruppe sind weniger günstig. In bevorzugter Weise werden daher $C_1$-$C_3$-Alkohole, wie Methanol, Ethanol, Propanol oder Isopropanol, bevorzugt Methanol, eingesetzt. Die Menge des Lösungsmittels wird bevorzugt so gewählt, daß der Reaktionsansatz während der Reaktion vollständig flüssig ist. Dies hängt in einer, dem Fachmann bekannten Weise, auch von der gewählten Reaktionstemperatur ab. Die Menge des alkoholischen Lösungsmittels beträgt im allgemeinen 10-80 Gew.-%, bevorzugt 15-60 Gew.-%, besonders bevorzugt 20-40 Gew.-%, des gesamten Reaktionsgemisches.

Die Reaktionstemperatur kann prinzipiell in einem weiten Bereich gewählt werden. Bei tieferen Tempera-

2

turen, beispielsweise bis hinunter zum Erstarrungspunkt des Lösungsmittels besteht jedoch die Gefahr, daß der Ansatz teilweise auskristallisiert; bei höheren Temperaturen besteht die Gefahr der Nebenproduktbildung. In bevorzugter Weise wird daher bei 50-85°C, besonders bevorzugt bei 60-75°C, gearbeitet.

Die Kondensation des Pinakolons mit p-Chlorbenzaldehyd wird beispielsweise so durchgeführt, daß das Lösungsmittel mit der gewünschten Base und einem der beiden Reaktionspartner vorgelegt wird und der andere Reaktionspartner zudosiert wird. In bevorzugter Weise wird die gesamte Menge an Pinakolon vorgelegt und der p-Chlorbenzaldehyd zudosiert. Nach dem Zusatz des p-Chlorbenzaldehyds wird in bevorzugter Weise noch eine Nachreaktionszeit von 1-3 Stunden angesetzt. Die so gewonnene Reaktionsmischung aus der Kondensation wird in vollständig flüssiger Form der Hydrierung zugeführt. Sollte es zur Aufrechterhaltung des vollständig flüssigen Zustandes erforderlich sein, weiteres Lösungsmittel zuzusetzen, wählt man in bevorzugter Weise das gleiche Lösungsmittel wie für die Kondensation. Auch nach dem Zusatz von weiteren Lösungsmitteln soll dessen Anteil am Reaktionsgemisch nicht über 80 Gew.-%, bevorzugt nicht über 60 Gew.-%, betragen.

Die Hydrierung kann im alkalischen Reaktionsgemisch oder im zuvor ganz oder teilweise neutralisierten Reaktionsgemisch vorgenommen werden. Bevorzugt ist es, soviel Säure zuzusetzen, daß 50-75 % der vorhandenen Base neutralisiert werden. Als Säuren können anorganische Säuren, wie Salzsäure, Salpetersäure, Schwefelsäure, Phosphorsäure oder Kohlensäure oder organische Säuren, wie Ameisensäure, Essigsäure, Propionsäure oder Oxalsäure, eingesetzt werden.

Als Hydrierkatalysatoren sind prinzipiell alle literaturbekannten Katalysatoren geeignet, soweit sie nicht Nebenreaktionen hervorrufen. In bevorzugter Weise wird Raney-Nickel gewählt, dem zur weiteren Vermeidung von Nebenreaktionen Verbindungen des zweiwertigen Schwefels, bevorzugt Thiodiglykol (Bis-(2-hydroxyethyl)-sulfid), zugesetzt werden können. Die Menge an Hydrierkatalysator wird im allgemeinen mit 1-10 Gew.-%, bevorzugt 4-6 Gew.-%, bezogen auf das Kondensationsprodukt, gewählt. Die zweiwertige Schwefelverbindung wird mit etwa 3-20 Gew.-%, bevorzugt 6-10 Gew.-%, bezogen auf den Hydrierkatalysator, gewählt.

Der Wasserstoffdruck beträgt erfindungsgemäß 5-500 bar, bevorzugt 10-400 bar, besonders bevorzugt 100-300 bar.

Die Hydriertemperatur wird von 50-130°C, bevorzugt von 80-120°C, gewählt.

Nach der Hydrierung wird das Reaktionsgemisch durch übliche Trennmethoden, wie Filtration, Dekantieren oder Zentrifugieren, vom Hydrierkatalysator befreit. Danach liegt im allgemeinen eine in allen Anteilen flüssige Hydriermischung vor. Von dieser Mischung wird erfindungsgemäß der Alkohol weitgehend abdestilliert. Diese Destillation wird so durchgeführt, daß der Destillationssumpf aufgrund seines Wassergehalts sich in eine wäßrige und eine organische Phase trennt. Das Wasser gelangt in das Reaktionsgemisch in form von Reaktionswasser bei der Kondensation, in Form von Verdünnungswasser der Base, in Form von Verdünnungswasser der zur vollständigen oder teilweisen Neutralisation eingesetzten Säure sowie in Form von Reaktionswasser bei der Neutralisation. Sollte der Wassergehalt nicht ausreichen, um die Trennung in zwei Phasen zu ermöglichen, kann weiteres Wasser nachgesetzt werden. Für den Fall, daß beim Destillieren des Alkohols Wasser aus dem Reaktionsgemisch entweicht, muß auch dieses Wasser ersetzt werden; dies ist insbesondere bei Alkohol/Wasser-Azeotropen erforderlich. Der Alkoholgehalt im Reaktionsgemisch beträgt nach der Destillation weniger als 2 Gew.-%, bevorzugt weniger als 0,5 Gew.-%, des gesamten Reaktionsgemisches. Der Wassergehalt des Destillationssumpfes ist insbesondere so groß, daß dieser Sumpf in allen Teilen (organische und wäßrige Phase) flüssig ist. Der Wasseranteil des Reaktionsgemisches wird so gehalten, daß er 5-30 Gew.-%, bevorzugt 7-15 Gew.-%, bezogen auf die Gesamtmenge an Reaktionsgemisch vor der Alkoholdestillation, beträgt.

Das nach der Alkoholdestillation vorliegende und in allen Teilen flüssige Gemisch trennt sich in eine wäßrige und eine organische Schicht. Beide Schichten können durch alle technisch üblichen Methoden voneinander getrennt werden, beispielsweise durch einfaches Absitzen in einem Behälter, durch die Benutzung einer Scheideflasche mit oder ohne Einbauten, die eine Phasentrennung unterstützen, oder durch Verwendung von Separatoren nach dem Zentrifugenprinzip.

Um das zu trennende Reaktionsgemisch in allen Teilen flüssig zu halten, wird die Phasentrennung bei einer Temperatur oberhalb der Raumtemperatur durchgeführt. Bevorzugt ist eine Temperatur oberhalb von 50°C, besonders bevorzugt oberhalb von 80°C. Die obere Temperaturgrenze ist durch den Siedepunkt der wäßrigen Phase gegeben, wobei es jedoch ungünstig ist, die Trennung in der Nähe dieses Siedepunktes durchzuführen. Die Obergrenze der Temperatur liegt daher bei maximal 100°C, bevorzugt bei maximal 90°C.

In der abgetrennten wäßrigen Phase befinden sich die wasserlöslichen Anteile der Reaktionsmischung. Dies sind die Ionen der zugesetzten Katalysatorbase der Kondensationsstufe und gegebenenfalls die der später zur vollständigen oder teilweisen Neutralisation zugesetzten Säure. Als Bestandteile der wäßrigen Phase kommen weiterhin saure Nebenbestandteile der Einsatzstoffe in Frage, ebenso wie das Anion der als Nebenprodukt entstandenen p-Chlorbenzoesäure. Diese p-Chlorbenzoesäure kann durch Ansäuern als weiteres Reaktionsprodukt isoliert werden.

3

Die organische Phase nach der Trennoperation besteht im wesentlichen aus 4,4-Dimethyl-1-(p-chlor-phenyl)-pentan-3-on. Durch einfaches Überdestillieren des Pentanons ist es zunächst möglich, alle Hochsieder zu entfernen; daran kann sich eine fraktionierte Destillation anschließen. Es ist jedoch auch möglich, direkt eine fraktioniert Destillation durchzuführen. Diese fraktionierte Destillation führt in einer dem Fachmann bekannten Weise zu jeder gewünschten Reinheitsstufe des Pentanons.

Erfindungsgemäß wird das 4,4-Dimethyl-1-(p-chlor-phenyl)-pentan-3-on in 90 % der theoretischen Ausbeute erhalten.

Das erfindungsgemäße Verfahren kann sowohl kontinuierlich, diskontinuierlich oder teilkontinuierlich ausgeführt werden. Das erfindungsgemäße Verfahren besitzt eine Reihe von unerwarteten Vorteilen gegenüber den bekannten Verfahrensweisen:

1. So ist es sehr vorteilhaft, die Zwischenisolierung des bei Raumtemperatur festen 4,4-Dimethyl-1-(p-chlor-phenyl)-1-penten-3-ons zu umgehen. Dies ist erfindungsgemäß durch den Einsatz der rohen Kondensationsmischung zur Hydrierung möglich.

2. Es ist durchaus überraschend, daß sich das rohe Pentenon in der angegebenen Form ohne wesentliche Nebenreaktionen hydrieren läßt.

3. Ein weiterer Vorteil des erfindungsgemäßen Verfahrens ist die beschriebene Phasentrennung in eine wäßrige und eine organische Phase. Hierbei wird die Katalysatorbase aus der Kondensationsstufe in verfahrenstechnisch günstiger Form ausgeschleust und weiterhin fast alle löslichen Verunreinigungen aus der organischen Phase genommen.

4. Es ist weiterhin ausgesprochen überraschend, daß sich die organische Phase nach der Trennoperation im wesentlichen unzersetzt destillieren läßt. Dies war nach dem bekannten Stand der Technik nicht zu erwarten, da eine Destillation der Gesamtmischung ohne Abtrennung der wäßrigen Phase zu Zersetzungserscheinungen im Destillationssumpf und zu einem starken Ausbeuteverlust führt (siehe Vergleichsbeispiel).

5. Ein beträchtlicher Vorteil des erfindungsgemäßen Verfahrens liegt darin, daß zu jedem Zeitpunkt nur flüssige Reaktionsgemische vorliegen, die verfahrenstechnisch gut zu handhaben sind.

6. Das 4,4-Dimethyl-1-(p-chlor-phenyl)-pentan-3-on wird in hoher Ausbeute und hoher Reinheit durch destillative Aufarbeitung erhalten.

Die folgenden Beispiele sollen das erfindungsgemäße Verfahren erläutern, ohne es jedoch auf diese einzuschränken.

## Beispiel 1

Bei Raumtemperatur wurden 1000 Gew.-Teile an Pinakolon von ca. 96 %iger Reinheit mit 1080 Gew.-Teilen Methanol und 100 Gew.-Teilen einer wäßrigen konzentrierten Natronlauge (ca. 48 %ig an NaOH) gemischt und unter Rühren auf 65-70°C erhitzt. Im Verlaufe von 2 h wurden 1320 Gew.-Teile p-Chlorbenzaldehyd als Schmelze zudosiert. Man rührte noch 2 h bei 65-70°C nach und gab 100 Gew.-Teile wasserfeuchtes Raney-Nickel (ca. 50 Gew.-Teile trockenes Raney-Nickel) und 5 Gew.-Teile Thiodiglykol zu und erhitzte unter Zufuhr von Wasserstoff mit einem Enddruck von 150 bar auf 80°C. Nach dem Ende der Wasserstoffaufnahme nach 20 Min. wurde der Katalysator abfiltriert und das Methanol über eine kurze Kolonne abdestilliert. Zu dem Sumpf wurden, bezogen auf die ehemaligen 1000 Gew.-Teile Pinakolon, 150 Gew.-Teile Wasser gegeben. Man gab die Mischung bei 80°C in einen Absetzbehälter, die untere wäßrige Phase wurde abgetrennt. Die organische Phase wurde über eine einfache Destillationseinrichtung destilliert.
Auswaage:     2000 Gew.-Teile, Reinheit: 96 %
Ausbeute:     91 % der theoretischen Ausbeute.
Zur Erhöhung der Reinheit kann über eine Kolonne destilliert werden.

## Beispiel 2

Das Verfahren von Beispiel 1 wurde mit folgenden Abänderungen wiederholt:
Nach der Zugabe von Raney-Nickel und Thiodiglykol wurde bei 10 bar statt 150 bar Wasserstoffdruck hydriert.
Dauer der Aufnahme: 160 Min.
Nach der Methanoldestillation wurden 160 Gew.-Teile Wasser zugegeben und das Gemisch bei 90°C im Absetzbehälter getrennt.
Auswaage:     1925 Gew.-Teile, Reinheit: 97,0 %
Ausbeute:     88,5 % der theoretischen Ausbeute

EP 0 355 350 B1

Beispiel 3

Das Verfahren von Beispiel 1 wurde mit folgender Abänderung wiederholt:
Vor der Zugabe des Hydrierkatalysators Raney-Nickel wurden 60 Gew.-Teile konzentrierte Salzsäure zugegeben.
Auswaage:    2015 Gew.-Teile, Reinheit: 96,3 %
Ausbeute:    92 % der theoretischen Ausbeute.

Beispiele 4 - 5

Das Verfahren von Beispiel 3 wurde wiederholt, man gab jedoch 60 Gew.Teile 50 %ige wäßrige Schwefelsäure zu.
Ausbeute: 89,3 % der theoretischen Ausbeute
Beispiel 3 wurde nochmal wiederholt, man gab jedoch 35 Gew.-Teile an Eisessig zu.
Ausbeute: 90,5 % der theoretischen Ausbeute.

Beispiel 6

Das Verfahren von Beispiel 1 wurde mit folgenden Abänderungen wiederholt:
Nach dem Abfiltrieren des Hydrierkatalysators wurden 200 Gew.-Teile (bezogen auf die ehemaligen 1000 Gew.-Teile Pinakolon) Wasser zugegeben.
Nach dem Abdestillieren des Lösungsmittels Methanol wurde das Gemisch durch Zentrifugieren bei ca. 65-75°C getrennt.
Auswaage:    1970 Gew.-Teile, Reinheit: 97,5 %
Ausbeute:    91 % der theoretischen Ausbeute.

Beispiel 7 (Vergleichsbeispiel)

Das Verfahren von Beispiel 1 wurde mit folgender Abänderung wiederholt:
Nach der Destillation des Lösungsmittels Methanol wurde kein Wasser zugesetzt und die gesamte Mischung wurde über eine einfache Destillationseinrichtung destilliert.
Auswaage:    1750 Gew.-Teile, Reinheit: 93,4 %
Ausbeute:    77,5 % der theoretischen Ausbeute.

## Patentansprüche

1.  Verfahren zur Herstellung von 4,4-Dimethyl-1-(p-chlor-phenyl)-pentan-3-on durch Kondensation von Pinakolon und p-Chlorbenzaldehyd in einem Alkohol als Lösungsmittel in Gegenwart einer anorganischen Base und anschließende Hydrierung, dadurch gekennzeichnet, daß man
    a) das bei der Kondensation erhaltene Reaktionsgemisch direkt ohne Zwischenisolierung des 4,4-Dimethyl-1-(p-chlor-phenyl)-1-penten-3-ons nach Zugabe eines Hydrierkatalysators bei erhöhter Temperatur und überatmosphärischem Druck zum 4,4-Dimethyl-1-(p-chlor-phenyl)-pentan-3-on hydriert,
    b) nach Abtrennung des Hydrierkatalysators vom flüssigen Hydriergemisch den Alkohol weitgehend abdestilliert und den Wassergehalt des Destillationssumpfes so einstellt, daß er sich in eine wäßrige und eine organische Phase trennt und
    c) das 4,4-Dimethyl-1-(p-chlor-phenyl)-pentan-3-on aus der organischen Phase gewinnt.

2.  Verfahren nach Anspruch 1, dadurch gekennzeichnet, daß man Hydroxide oder Carbonate von Alkali- oder Erdalkali-Metallen als Base einsetzt.

3.  Verfahren nach Anspruch 1, dadurch gekennzeichnet, daß man die Kondensation von Pinakolon und p-Chlorbenzaldehyd bei 50-85°C durchführt.

4.  Verfahren nach Anspruch 1, dadurch gekennzeichnet, daß der Alkohol Methanol ist.

5.  Verfahren nach Anspruch 1, dadurch gekennzeichnet, daß man nach der Kondensation so viel Säure zugibt, daß die gesamte Menge der in der Mischung enthaltenen Base zu 50-75 % neutralisiert wird.

5

**6.** Verfahren nach Anspruch 1, dadurch gekennzeichnet, daß man bei einem Wasserstoffdruck von 5-500 bar hydriert.

**7.** Verfahren nach Anspruch 1, dadurch gekennzeichnet, daß man bei Temperaturen von 50-130°C hydriert.

**8.** Verfahren nach Anspruch 1, dadurch gekennzeichnet, daß das hydrierte Reaktionsgemisch vor der Alkoholdestillation 10-30 Gew.-% Wasser, bezogen auf die Gesamtmenge der Mischung, enthält.

**9.** Verfahren nach Anspruch 1, dadurch gekennzeichnet, daß man nach der Abtrennung des Hydrierkatalysators die Phasentrennung bei 50-100°C durchführt.

**10.** Verfahren nach Anspruch 9, dadurch gekennzeichnet, daß man die Phasentrennung durch kontinuierliches Zentrifugieren in einem Separator durchführt.

## Claims

**1.** Process for the preparation of 4,4-dimethyl-1-(p-chlorophenyl)pentan-3-one by condensation of pinacolone and p-chlorobenzaldehyde in an alcohol as solvent in the presence of an inorganic base and subsequent hydrogenation which is characterized in that

a) the reaction mixture obtained in the condensation is hydrogenated directly without isolation of the intermediate 4,4-dimethyl-1-(p-chlorophenyl)-1-penten-3-one after the addition of a hydrogenation catalyst at elevated temperature and super-atmospheric pressure to give 4,4-dimethyl-1-(p-chlorophenyl)pentan-3-one,

b) after the hydrogenation catalyst is separated off from the liquid hydrogenation mixture, the alcohol is largely distilled off, and the water content of the bottom product of the distillation is adjusted in such a manner that it separates into an aqueous and an organic phase and

c) 4,4-dimethyl-1-(p-chlorophenyl)pentan-3-one is recovered from the organic phase.

**2.** Process according to Claim 1, characterized in that hydroxides or carbonates of alkali metals or alkaline earth metals are used as the base.

**3.** Process according to Claim 1, characterized in that the condensation of pinacolone and p-chlorobenzaldehyde is carried out at 50-85°C.

**4.** Process according to Claim 1, characterized in that the alcohol is methanol.

**5.** Process according to Claim 1, characterized in that after the condensation such an amount of acid is added that 50-75% of the entire amount of the base contained in the mixture is neutralized.

**6.** Process according to Claim 1, characterized in that the hydrogenation is carried out at a hydrogen pressure of 5-500 bar.

**7.** Process according to Claim 1, characterized in that the hydrogenation is carried out at temperatures of 50-130°C.

**8.** Process according to Claim 1, characterized in that before the distillation of the alcohol the hydrogenated reaction mixture contains 10-30% by weight of water, relative to the total amount of the mixture.

**9.** Process according to Claim 1, characterized in that, after the hydrogenation catalyst has been separated off, the phase separation is carried out at 50-100°C.

**10.** Process according to Claim 9, characterized in that the phase separation is carried out by continuous centrifugation in a separator.

## Revendications

**1.** Procédé de préparation de 4,4-diméthyl-1-(p-chlorophényl)pentane-3-one par condensation de pinacolone et de p-chlorobenzaldéhyde dans un alcool comme solvant, en présence d'une base inorganique, et hydrogénation subséquente, caractérisé en ce que

    a) on hydrogène directement le mélange réactionnel obtenu lors de la condensation, sans isolement intermédiaire de la 4,4-diméthyl-1-(p-chlorophényl)-1-pentène-3-one, après avoir ajouté un catalyseur d'hydrogénation, à une température élevée et sous une pression supérieure à la pression atmosphérique, pour donner la 4,4-diméthyl-1-(p-chlorophényl)pentane-3-one,

    b) après avoir séparé le catalyseur d'hydrogénation du mélange d'hydrogénation liquide, on sépare par distillation une grande partie de l'alcool et on ajuste la teneur en eau du résidu de distillation de façon à ce qu'il se sépare en une phase aqueuse et une phase organique et

    c) on récupère la 4,4-diméthyl-(p-chlorophényl)pentane-3-one à partir de la phase organique.

2. Procédé selon la revendication 1, caractérisé en ce qu'on utilise comme base des hydroxydes ou des carbonates de métaux alcalins ou alcalino-terreux.

3. Procédé selon la revendication 1, caractérisé en ce qu'on effectue la condensation de la pinacolone et du p-chlorobenzaldéhyde à 50-85°C.

4. Procédé selon la revendication 1, caractérisé en ce que l'alcool est du méthanol.

5. Procédé selon la revendication 1, caractérisé en ce que, après la condensation, on ajoute un acide en une quantité telle que la quantité totale de la base contenue dans le mélange soit neutralisée à 50-75 %.

6. Procédé selon la revendication 1, caractérisé en ce qu'on hydrogène sous une pression d'hydrogène de 5-500 bars.

7. Procédé selon la revendication 1, caractérisé en ce qu'on hydrogène à des températures de 50-130°C.

8. Procédé selon la revendication 1, caractérisé en ce que le mélange réactionnel hydrogéné contient, avant la distillation de l'alcool, 10 à 30 % en poids d'eau par rapport à la quantité totale du mélange.

9. Procédé selon la revendication 1, caractérisé en ce que, après avoir séparé le catalyseur d'hydrogénation, on effectue la séparation de phases à 50-100°C.

10. Procédé selon la revendication 9, caractérisé en ce qu'on effectue la séparation de phases par centrifugation continue dans un séparateur.